# FASCICULE DE BREVET EUROPEEN

(11) **EP 1 000 609 B1**
(45) Date de publication et mention de la délivrance du brevet: **03.07.2002**
(21) Numéro de dépôt: 99402567.4
(22) Date de dépôt: 18.10.1999
(51) Int. Cl.: A61K 7/043, A61K 7/48

(54) **Utilisation d'un ester de cellulose pour diminuer le temps de séchage d'une composition de vernis à ongles contenant de la nitrocellulose**
Verwendung von einem Zellulose-Ester zur Verminderung des Trocknungszeit eines Nagellacks auf Basis von Nitrozellulose
Use of a cellulose ester for decreasing the drying time of a nail varnish composition containing nitrocellulose

(30) Priorité: 06.11.1998 FR 9814111
(43) Date de publication de la demande: 17.05.2000
(73) Titulaire: L'OREAL, 75008 Paris (FR)
(72) Inventeur: Ramin, Roland, 75014 Paris (FR)
(74) Mandataire: Lhoste, Catherine

(56) Documents cités:
- EP-A- 0 432 572
- WO-A-93/18098
- US-A- 5 607 665
- US-A- 5 639 447

## Description

L'invention a pour objet l'utilisation d'un ester de cellulose dans une composition de vernis à ongles comprenant de la nitrocellulose pour diminuer le temps de séchage de la composition.

Les compositions de vernis à ongles comprennent généralement un polymère filmogène solubilisé dans un milieu solvant organique. Parmi les polymères filmogènes les plus couramment utilisés dans ces compositions, on peu citer la nitrocellulose. Ces compositions sont généralement appliquées en une ou plusieurs couches sur la surface de l'ongle, conduisant après séchage à la formation d'un film sur l'ongle.

Les couches de vernis à ongles déposées peuvent toutefois, juste après leur application, être endommagées ou dégradées par contact, friction ou accrochage sur des corps étrangers comme par exemple les vêtements. Aussi, il est souhaitable que ces vernis sèchent rapidement après leur application.

Dans l'art antérieur, il est connu d'améliorer la vitesse de séchage en sélectionnant des solvants appropriés. Par exemple, le document US-A-5424061 propose l'emploi d'acétone associé à un autre cosolvant. Cette sélection a pour contrainte une limitation de la formulation du vernis à ongles. De plus, des solvants trop volatils peuvent nuire à la qualité du maquillage obtenu. En effet, des défauts peuvent apparaître lors de l'application du vernis à ongles : formation de stries sur la couche de vernis, séchage trop rapide du pinceau lors de l'application. On obtient ainsi un film non homogène et pas suffisamment lisse ; il est peu brillant et présente une tenue non satisfaisante.

Pour améliorer la vitesse de séchage du vernis, il est également connu d'employer des additifs particuliers comme par exemple des composés siliconés qui sont décrit dans le document EP-A-432572.

Par ailleurs, il est connu d'associer à la nitrocellulose d'autres polymères filmogènes dans le but de modifier les propriétés cosmétiques du film de vernis à ongles, comme le montrent les exemples du document EP-A-432572.
Il est également connu du document WO-A-93/18098 un vernis à ongles comprenant de la nitrocellulose et de l'acétopropionate de cellulose. Les propriétés de séchage de ce vernis ne sont nullement décrites. Le document US-A-5639447 décrit un vernis à ongles séchant en moins de 70 secondes comprenant un mélange de deux polymères filmogènes.

Le but de la présente invention est de proposer une composition de vernis à ongles à base de nitrocellulose ayant des propriétés de séchage améliorée sans employer des solvants ou additifs particuliers.

Le demandeur a découvert que l'association de nitrocellulose et d'esters de cellulose, dans certaines proportions, permettait d'obtenir un vernis à ongles dont le temps de séchage est inférieur au temps de séchage d'une composition similaire ne contenant que la nitrocellulose ou que l'ester de cellulose comme polymère filmogène.

Contrairement à l'enseignement de l'exemple 4 du document EP-A-432572 qui décrit un vernis à ongles présentant de mauvaise propriété de séchage et comprenant de la nitrocellulose, une résine alkyde et de l'acétobutyrate de cellulose, le demandeur a constaté que l'association de nitrocellulose et d'acétobutyrate de cellulose en quantité particulière permettait d'obtenir une synergie du temps de séchage de la composition.

De façon plus précise, l'invention a pour objet l'utilisation d'un ester de cellulose dans une composition de vernis à ongles comprenant; dans un milieu solvant, un système polymérique contenant de la nitrocellulose et de 0 % à 50 % en poids, par rapport au poids total de nitrocellulose et d'ester de cellulose, de polymère filmogène auxiliaire, et une matière colorante en quantité suffisante pour colorer la composition, l'ester de cellulose étant présent en une quantité efficace et synergique, par rapport à la quantité de nitrocellulose, pour diminuer le temps de séchage de la composition.

L'ester de cellulose utilisé selon l'invention peut comprendre des groupements acyle R-CO- dont le groupement R désigne un radical alkyle, linéaire ou ramifié, ayant de 1 à 3 atomes de carbones, ou leurs mélanges.

En particulier, l'ester de cellulose peut être choisi parmi l'acétate de cellulose, l'acétopropionate de cellulose, l'acétobutyrate de cellulose, et de préférence parmi l'acétobutyrate de cellulose et l'acétopropionate de cellulose.

L'acétobutyrate de cellulose peut être présent dans la composition de vernis à ongle en une teneur pondérale allant de 10 % à 80 % en poids, par rapport au poids total d'ester de cellulose et de nitrocellulose.

L'acétobutyrate de cellulose peut comprendre un taux pondéral en groupement acétate allant de 1 à 18 % et un taux pondéral en groupement butyrate allant de 30 à 60 %. Comme acétobutyrate de cellulose (dit ABC), on peut utiliser ceux vendus sous les dénominations "CAB-551", "CAB-500", "CAB 553", "CAB-381"par la société Eastman Chemical.

Les taux pondéraux en groupement acétate et butyrate de ces composés est la suivante:

| **Produit ABC** | **Taux acétate en %** | **Taux butyrate en %** |
|---|---|---|
| CAB-551 | 2 | 53 |
| CAB-500 | 5 | 49 |
| CAB 553 | 2 | 47 |
| CAB-381 | 13 | 37 |

Lorsque le taux pondéral en groupement acétate inférieur ou égal à 5 %, l'acétobutyrate de cellulose est de préférence présent en une. teneur pondérale allant de 10 % à 30 % en poids, et mieux de 12,5 % à 25 % en poids, par rapport au poids total d'ester de cellulose et de nitrocellulose.

Lorsque le taux pondéral en groupement acétate est supérieur ou égal à 10 %, l'acétobutyrate de cellulose est de préférence présent en une teneur pondérale allant de 20 % à 80 % en poids, et mieux de 25 % à 75 % en poids, par rapport au poids total d'ester de cellulose et de nitrocellulose présents dans la composition.

L'acétoproprionate de cellulose peut être présent dans la composition de vernis à ongles en une teneur pondérale allant de 10 % à 80 % en poids, et mieux de 12,5 % à 75 % en poids, par rapport au poids total d'ester de cellulose et de nitrocellulose présents dans la composition.

De préférence, l'acétopropionate de cellulose peut comprendre un taux pondéral en groupement acétate allant de 1 à 5 % et un taux pondéral en groupement propionate allant de 35 à 50 %. On peut utiliser les acétopropionates de cellulose (dit APC) vendus sous les dénominations "CAP-482-0,5", "CAP-482-20" et "CAP-504" par la société Eastman Chemical.

Les taux pondéraux en groupement acétate et propionate de ces composés est la suivante:

| **Produit APC** | **Taux acétate en %** | **Taux propionate en %** |
|---|---|---|
| CAP-482-0,5 | 2,5 | 44 |
| CAP-482-20 | 2,5 | 46 |
| CAB 504 | 2,5 | 40 |

La nitrocellulose utilisée selon l'invention peut être choisie parmi la nitrocellulose RS 1/8 sec ; RS ¼ sec. ; ½ sec. ; RS 5 sec. ; RS 15 sec. ; RS 35 sec. ; RS 75 sec.; RS 150 sec ; AS ¼ sec. ; AS ½ sec. ; SS ¼ sec. ; SS ½ sec. ; SS 5 sec., notamment commercialisée par la société HERCULES.

Avantageusement, la composition pour l'utilisation selon l'invention peut comprendre une teneur en nitrocellulose allant de 1,5 à 35 % en poids, par rapport au poids total de la composition, et mieux de 8 % à 20 % en poids.

Selon un premier mode de réalisation selon l'invention, la composition ne comprend que la nitrocellulose et l'ester de cellulose tels que définis précédemment comme polymères filmogènes.

Selon un deuxième mode de réalisation selon l'invention, la composition pour l'utilisation selon l'invention peut comprendre, en outre, un polymère filmogène auxiliaire, notament pour améliorer les propriétés cosmétiques et physico-chimiques du film de vernis à ongles.

Avantageusement, le système polymérique présent dans la composition peut contenir moins de 40 % en poids, par rapport au poids total de nitrocellulose et d'ester de cellulose, de polymère filmogène auxiliaire.

Le polymère filmogène auxiliaire peut être choisi parmi les butyralpolyvinyliques, les résines alkydes, les résines résultant de la condensation de formaldéhyde avec une arylsulfonamide, les polyesters, les polyuréthannes, les polyester-polyuréthanes, les polyéther-polyuréthanes, les polymères radicalaires, notamment de type acrylique, acrylique-styrène et/ou vinylique et leurs mélanges.

Le polymère filmogène auxiliaire peut être présent dans la composition de vernis à ongles en une teneur allant de 0 % à 50% en poids, par rapport à au poids total de nitrocellulose et d'ester de cellulose, et mieux de 1 % à 15 % en poids.

Le milieu solvant de la composition de vernis à ongles peut comprendre au moins un solvant organique.

Comme solvant organique utilisable dans l'invention, on peut citer :
- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les composés cycliques aromatiques liquides à température ambiante tels que le toluène et le xylène ;
- les aldéhydes liquides à température ambiante tels que le benzaldéhyde, l'acétaldéhyde
- leurs mélanges.

La composition pour l'utilisation selon l'invention peut comprendre en outre de l'eau, notamment en une teneur allant de 0 à 10 % en poids, par rapport au poids total de la composition.

La composition peut comprendre, en outre, au moins un agent plastifiant. En particulier, on peut citer, seuls ou en mélange, les plastifiants usuels, tels que:
- les glycols et leurs dérivés tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther;
- les esters de glycérol,
- les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther,
- des esters d'acides notamment carboxyliques, tels que des citrates, des phtalates, des adipates, des carbonates, des tartrates, des phosphates, des sébaçates,
- des dérivés oxyéthylénés tels que les huiles oxyéthylénées, notamment les huiles végétales telles que l'huile de ricin;
- leurs mélanges.

La quantité de plastifiant peut être choisie par l'homme du métier sur base de ses connaissances générales, de manière à obtenir une composition ayant des propriétés cosmétiquement acceptables. La teneur en plastifiant peut par exemple aller de 0,5 % à 20 % en poids, par rapport au poids total de la composition, et de préférence de 2 % à 10 % en poids.

La matière colorante présente dans la composition peut être choisie parmi les composés pulvérulents et/ou les colorants solubles dans le milieu solvant de la composition, en une quantité suffisante pour colorer le vernis en une couleur du domaine de longueur d'onde visible, à savoir entre 400 et 800 nm, et par exemple en une teneur allant de 0,001 % à 10 % en poids, par rapport au poids total de la composition. Les composés pulvérulents peuvent être choisis parmi les pigments et/ou les nacres et/ou les paillettes habituellement utilisés dans les vernis à ongles.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique, les pigments métalliques comme l'aluminium ou le bronze. Parmi les pigments organiques, on peut citer le noir de carbone, les pigments de type D & C, et les laques à base de carmin de cochenille, de baryum, strontium, calcium, aluminium, la guanine.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les paillettes peuvent être choisies parmi celles en résine acrylique, polyester, polyéthylène téréphtalate, en aluminium.

Les colorants sont par exemple le rouge Soudan, le DC Red 17, le DC Green 6, le β-carotène, l'huile de soja, le brun Soudan, le DC Yellow, 11, le DC Violet 2, le DC orange 5, le jaune quinoléine.

La composition de l'invention peut éventuellement comprendre un azurant optique, par exemple pour diminuer le jaunissement de la nitrocellulose et par conséquent de la composition, cet azurant n'étant pas considérer, au sens de l'invention, comme une matière colorante. Comme azurant, on peut citer le sel monosodique de pourpre d'alizurol ; il peut être présent en une teneur allant de 10⁻⁷ % à 10⁻⁴ % en poids, par rapport au poids total de la composition.

La composition pour l'utilisation selon l'invention peut en outre comprendre tout additif connu de l'homme du métier comme étant susceptible d'être incorporé dans une telle composition, tels que les agents épaississants, les agents d'étalement, les agents mouillants, les agents dispersants, les anti-mousses, les conservateurs, les filtres UV, les actifs, les tensioactifs, les agents hydratants, les parfums, les neutralisants, les stabilisants, les antioxydants. Bien entendu, l'homme du métier veillera à choisir ce ou ces éventuels composés complémentaires, et/ou leur quantité, de manière telle que les propriétés avantageuses de la composition pour l'utilisation selon l'invention ne soient pas, ou substantiellement pas, altérées par l'adjonction envisagée. Avantageusement, la composition est exempte d'agent de séchage siliconé.

La composition pour l'utilisation selon l'invention peut être préparée par l'homme du métier sur la base de ses connaissances générales et selon l'état de la technique.

L'invention est illustrée plus en détail dans les exemples suivants.

Le temps de séchage des compositions a été évalué dans les conditions suivantes

On dépose sur une plaque de verre chauffée à 30 °C un film de composition d'une épaisseur de 300 µm et on détermine, à l'aide de l'appareil Beck et Koller, le temps de séchage apparent du film. On a effectué 4 mesures et calculé le temps de séchage moyen exprimé en minute.

### Exemples 1 à 24 :

On a préparé 24 compositions non colorées suivantes :
- Nitrocellulose ½ sec. x g
- Ester de cellulose y g
- Acétyl tributyl citrate 4,5 g
- N-éthyl, o,p-toluène sulfonamide 3,6 g
- acétate d'éthyle qsp 100 g
avec x + y = 20 g

| a) Ester de cellulose : Acétobutyrate de cellulose 381-0,5 d'Eastman Chemical | | | |
|---|---|---|---|
| **Exemple** | **Nitrocellulose x** | **ABC 381-0,5 y** | **Temps de séchage** |
| 1 (HI) | 20 | 0 | 3,1 |
| 2 (HI) | 17,5 | 2,5 | 3,1 |
| 3 | 15 | 5 | 2,35 |
| 4 | 10 | 10 | 2,4 |
| 5 | 5 | 15 | 2,7 |
| 6 (HI) | 0 | 20 | 3,5 |
| HI signifie hors invention ABC signifie acétobutyrate de cellulose | | | |

## Revendications

1. Utilisation d'un ester de cellulose dans une composition de vernis à ongles comprenant, dans un milieu solvant, un système polymérique contenant de la nitrocellulose et de 0 % à 50 % en poids, par rapport au poids total de nitrocellulose et d'ester de cellulose, de polymère filmogène auxiliaire, et une matière colorante en quantité suffisante pour colorer la composition, l'ester de cellulose étant présent en une quantité efficace et synergique, par rapport à la quantité de nitrocellulose, pour obtenir un temps de séchage de la composition inférieur au temps de séchage d'une composition similaire ne contenant que la nitrocellulose ou que ledit ester de cellulose comme polymère filmogène à la place de l'association de nitrocellulose et d'ester de cellulose.

2. Utilisation selon la revendication 1, **caractérisée par le fait que** l'ester de cellulose comprend des groupements acyle R-CO- dont le groupement R désigne un radical alkyle, linéaire ou ramifié, ayant de 1 à 3 atomes de carbones, ou leurs mélanges.

3. Utilisation selon la revendication 1 ou 2, **caractérisée par le fait que** l'ester de cellulose est choisi dans le groupe formé par l'acétate de cellulose, l'acétopropionate de cellulose, l'acétobutyrate de cellulose.

4. Utilisation selon l'une quelconque des revendications précédentes, **caractérisée par le fait que** l'ester de cellulose est l'acétobutyrate de cellulose.

5. Utilisation selon l'une des revendications 3 ou 4, **caractérisée par le fait que** l'acétobutyrate de cellulose est présent en une teneur pondérale allant de 10 % à 80 % en poids, par rapport au poids total d'ester de cellulose et de nitrocellulose.

6. Utilisation selon l'une quelconque des revendications 3 à 5, **caractérisée par le fait que** l'acétobutyrate de cellulose comprend un taux pondéral en groupement acétate allant de 1 à 18 % et un taux pondéral en groupement butyrate allant de 30 à 60 %.

7. Utilisation selon la revendication 6, **caractérisée par le fait que** l'acétobutyrate de cellulose comprend un taux pondéral en groupement acétate inférieur ou égal à 5 % et est présent en une teneur pondérale allant de 10 % à 30 % en poids, par rapport au poids total d'ester de cellulose et de nitrocellulose.

8. Utilisation selon la revendication 6, **caractérisée par le fait que** l'acétobutyrate de cellulose comprend un taux pondéral en groupement acétate supérieur ou égal à 10 % et est présent en une teneur pondérale allant de 20 % à 80 % en poids, par rapport au poids total d'ester de cellulose et de nitrocellulose.

9. Utilisation selon l'une quelconque des revendications 1 à 3, **caractérisée par le fait que** l'ester de cellulose est l'acétoproprionate de cellulose.

10. Utilisation selon la revendication 9, **caractérisée par le fait que** l'acétoproprionate de cellulose est présent en une teneur pondérale allant de 10 % à 80 % en poids, par rapport au poids total d'ester de cellulose et de nitrocellulose.

11. Utilisation selon la revendication 9 ou 10, **caractérisée par le fait que** l'acétopropionate de cellulose comprend un taux pondéral en groupement acétate allant de 1 à 5 % et un taux pondéral en groupement propionate allant de 35 à 50 %.

12. Utilisation selon l'une quelconque des revendications précédentes, **caractérisée par le fait que** la composition comprend une teneur en nitrocellulose allant de 1,5 à 35 % en poids, par rapport au poids total de la composition, et mieux de 8 % à 20% en poids.

13. Utilisation selon l'une quelconque des revendications précédentes, **caractérisée par le fait que** la composition ne comprend que la nitrocellulose et l'ester de cellulose comme polymères filmogènes.

14. Utilisation selon l'une quelconque des revendications précédentes, **caractérisée par le fait que** le polymère filmogène auxiliaire est choisi dans le groupe formé par les butyralpolyvinyliques, les résines alkydes, les résines résultant de la condensation de formaldéhyde avec une arylsulfonamide, les polyesters, les polyuréthannes, les polyester-polyuréthanes, les polyéther-polyuréthanes, les polymères radicalaires, notamment de type acrylique, acrylique styrène et/ou vinylique.

15. Utilisation selon l'une quelconque des revendications précédentes, **caractérisée par le fait que** le polymère filmogène auxiliaire est présent en une teneur allant de 1 % à 15 % en poids, par rapport à au poids total de nitrocellulose et d'ester de cellulose.

16. Utilisation selon l'une quelconque des revendications précédentes, **caractérisée par le fait que** la matière colorante est choisie dans le groupe formé par les colorants solubles dans le milieu solvant de la composition, les pigments et les paillettes.

17. Utilisation selon l'une quelconque des revendications précédentes, **caractérisée par le fait que** la matière colorante est présente en une teneur allant de 0,001 % à 10 % en poids, par rapport au poids total de la composition.

18. Utilisation selon l'une quelconque des revendications précédentes, **caractérisée par le fait que** le milieu solvant comprend au moins un solvant organique.

19. Utilisation selon l'une quelconque des revendications précédentes, **caractérisée par le fait que** la composition comprend, en outre, au moins un agent plastifiant.

20. Utilisation selon l'une quelconque des revendications précédentes, **caractérisée par le fait que** la composition est exempte d'agent de séchage siliconé.

## Patentansprüche

1. Verwendung eines Celluloseesters in einer Nagellackzusammensetzung, die in einem Lösungsmittelmedium ein Polymersystem, das Nitrocellulose und 0 bis 50 Gew.-% eines zusätzlichen filmbildenden Polymers, bezogen auf das Gesamtgewicht der Nitrocellulose und des Celluloseesters, umfaßt, und ein Farbmittel in einer Menge enthält, die ausreichend ist, um die Zusammensetzung zu färben, wobei der Celluloseester, bezogen auf die Menge der Nitrocellulose, in einer Menge vorliegt, die synergistisch und wirksam ist, um eine Trocknungszeit der Zusammensetzung zu erhalten, die unter der Trocknungszeit einer ähnlichen Zusammensetzung liegt, die als filmbildendes Polymer anstelle der Kombination der Nitrocellulose und des Celluloseesters nur die Nitrocellulose oder nur den Celluloseester enthält.

2. Verwendung nach Anspruch 1, **dadurch gekennzeichnet, daß** der Celluloseester Acylgruppen R-CO-, wobei die Gruppe R eine geradkettige oder verzweigte Alkylgruppe mit 1 bis 3 Kohlenstoffatomen bedeutet, oder deren Gemische enthält.

3. Verwendung der Ansprüche 1 oder 2, **dadurch gekennzeichnet, daß** der Celluloseester unter Celluloseacetat, Celluloseacetopropionat und Celluloseacetobutyrat ausgewählt ist.

4. Verwendung nach einem der vorhergehenden Ansprüche, **dadurch gekennzeichnet, daß** der Celluloseester das Celluloseacetobutyrat ist.

5. Verwendung nach einem der Ansprüche 3 oder 4, **dadurch gekennzeichnet, daß** das Celluloseacetobutyrat in einer Gewichtsmenge von 10 bis 80 Gew.-%, bezogen auf das Gesamtgewicht der Nitrocellulose und des Celluloseesters, vorliegt.

6. Verwendung nach einem der Ansprüche 3 bis 5, **dadurch gekennzeichnet, daß** das Celluloseacetobutyrat einen Gewichtsanteil an Acetatgruppen von 1 bis 18 % und einen Gewichtsanteil an Butyratgruppen im Bereich von 30 bis 60 % enthält.

7. Verwendung nach Anspruch 6, **dadurch gekennzeichnet, daß** das Celluloseacetobutyrat einen Gewichtsanteil an Acetatgruppen von 5 % oder darunter aufweist und in einem Gewichtsanteil von 10 bis 30 Gew.-%, bezogen auf das Gesamtgewicht der Nitrocellulose und des Celluloseesters, vorliegt.

8. Verwendung nach Anspruch 6, **dadurch gekennzeichnet, daß** das Celluloseacetobutyrat einen Gewichtsanteil an Acetatgruppen von 10 % oder darüber aufweist und in einem Gewichtsanteil von 20 bis 80 Gew.-%, bezogen auf das Gesamtgewicht der Nitrocellulose und des Celluloseesters, vorliegt.

9. Verwendung nach einem der Ansprüche 1 bis 3, **dadurch gekennzeichnet, daß** der Celluloseester das Celluloseacetopropionat ist.

10. Verwendung nach Anspruch 9, **dadurch gekennzeichnet, daß** das Celluloseacetopropionat in einer Gewichtsmenge von 10 bis 80 Gew.-%, bezogen auf das Gesamtgewicht der Nitrocellulose und des Celluloseesters, vorliegt.

11. Verwendung nach Anspruch 9 oder 10, **dadurch gekennzeichnet, daß** das Celluloseacetopropionat einen Gewichtsanteil an Acetatgruppen von 1 bis 5 % und einen Gewichtsanteil an Propionatgruppen im Bereich von 35 bis 50 % enthält.

12. Verwendung nach einem der vorhergehenden Ansprüche, **dadurch gekennzeichnet, daß** die Zusammensetzung die Nitrocellulose in einem Mengenanteil von 1,5 bis 35 Gew.-%, bezogen auf das Gesamtgewicht der Zusammensetzung, und besser 8 bis 20 Gew.-% enthält.

13. Verwendung nach einem der vorhergehenden Ansprüche, **dadurch gekennzeichnet, daß** die Zusammensetzung nur die Nitrocellulose und den Celluloseester als filmbildende Polymere enthält.

14. Verwendung nach einem der vorhergehenden Ansprüche, **dadurch gekennzeichnet, daß** das zusätzliche filmbildende Polymer unter den Polyvinylbutyralen, Alkydharzen, Harzen, die bei der Kondensation von Formaldehyd und einem Arylsulfonamid entstehen, Polyestern, Polyurethanen, Polyester-Polyurethanen, Polyether-Polyurethanen und durch radikalische Polymerisation hergestellten Polymeren insbesondere vom Acryltyp, Acryl-Styroltyp und/oder Vinyltyp ausgewählt ist.

15. Verwendung nach einem der vorhergehenden Ansprüche, **dadurch gekennzeichnet, daß** das zusätzliche film bildende Polymer in einer Menge von 1 bis 15 Gew.-%, bezogen auf das Gesamtgewicht der Nitrocellulose und des Celluloseesters, vorliegt.

16. Verwendung nach einem der vorhergehenden Ansprüche, **dadurch gekennzeichnet, daß** das Farbmittel unter den in dem Lösungsmittelmedium der Zusammensetzung löslichen Farbmitteln, Pigmenten und Plättchen ausgewählt ist.

17. Verwendung nach einem der vorhergehenden Ansprüche, **dadurch gekennzeichnet, daß** das Farbmittel in einer Menge von 0,001 bis 10 Gew.-%, bezogen auf das Gesamtgewicht der Zusammensetzung, vorliegt.

18. Verwendung nach einem der vorhergehenden Ansprüche, **dadurch gekennzeichnet, daß** das Lösungsmittelmedium mindestens ein organisches Lösungsmittel enthält.

19. Verwendung nach einem der vorhergehenden Ansprüche, **dadurch gekennzeichnet, daß** die Zusammensetzung ferner mindestens einen Weichmacher enthält.

20. Verwendung nach einem der vorhergehenden Ansprüche, **dadurch gekennzeichnet, daß** die Zusammensetzung kein siliconhaltiges Trocknungsmittel enthält.

## Claims

1. Use of a cellulose ester in a nail varnish composition comprising, in a solvent medium, a polymer system containing nitrocellulose and from 0% to 50% by weight, relative to the total weight of nitrocellulose and cellulose ester, of additional film-forming polymer, and a dyestuff in an amount which is sufficient to dye the composition, the cellulose ester being present in an effective and synergistic amount relative to the amount of nitrocellulose, in order to obtain a drying time of the composition which is shorter than the drying time of a similar composition containing only nitrocellulose or containing only the said cellulose ester as film-forming polymer in place of the combination of nitrocellulose and cellulose ester.

2. Use according to Claim 1, **characterized in that** the cellulose ester comprises acyl groups R-CO- in which the group R denotes a linear or branched alkyl radical containing from 1 to 3 carbon atoms, or mixtures thereof.

3. Use according to Claim 1 or 2, **characterized in that** the cellulose ester is chosen from the group formed by cellulose acetate, cellulose acetopropionate and cellulose acetobutyrate.

4. Use according to any one of the preceding claims, **characterized in that** the cellulose ester is cellulose acetobutyrate.

5. Use according to either of Claims 3 and 4, **characterized in that** the cellulose acetobutyrate is present in a weight content ranging from 10% to 80% by weight relative to the total weight of cellulose ester and nitrocellulose.

6. Use according to any one of Claims 3 to 5, **characterized in that** the cellulose acetobutyrate comprises a weight content of acetate group ranging from 1 to 18% and a weight content of butyrate group ranging from 30 to 60%.

7. Use according to Claim 6, **characterized in that** the cellulose acetobutyrate comprises a weight content of acetate group of less than or equal to 5% and is present in a weight content ranging from 10% to 30% by weight relative to the total weight of cellulose ester and nitrocellulose.

8. Use according to Claim 6, **characterized in that** the cellulose acetobutyrate comprises a weight content of acetate group of greater than or equal to 10% and is present in a weight content ranging from 20% to 80% by weight relative to the total weight of cellulose ester and nitrocellulose.

9. Use according to any one of Claims 1 to 3, **characterized in that** the cellulose ester is cellulose acetopropionate.

10. Use according to Claim 9, **characterized in that** the cellulose acetopropionate is present in a weight content ranging from 10% to 80% by weight relative to the total weight of cellulose ester and nitrocellulose.

11. Use according to Claim 9 or 10, **characterized in that** the cellulose acetopropionate comprises a weight content of acetate group ranging from 1 to 5% and a weight content of propionate group ranging from 35 to 50%.

12. Use according to any one of the preceding claims, **characterized in that** the composition comprises a nitrocellulose content ranging from 1.5 to 35% by weight relative to the total weight of the composition, and better still from 8% to 20% by weight.

13. Use according to any one of the preceding claims, **characterized in that** the composition comprises only nitrocellulose and cellulose ester as film-forming polymers.

14. Use according to any one of the preceding claims, **characterized in that** the additional film-forming polymer is chosen from the group formed by polyvinyl butyrals, alkyd resins, resins resulting from the condensation of formaldehyde with an arylsulphonamide, polyesters, polyurethanes, polyester-polyurethanes, polyether-polyurethanes and radical-generated polymers, in particular of acrylic, acrylic-styrene and/or vinyl type.

15. Use according to any one of the preceding claims, **characterized in that** the additional film-forming polymer is present in a content ranging from 1% to 15% by weight relative to the total weight of nitrocellulose and cellulose ester.

16. Use according to any one of the preceding claims, **characterized in that** the dyestuff is chosen from the group formed by dyes which are soluble in the solvent medium for the composition, pigments and glitter flakes.

17. Use according to any one of the preceding claims, **characterized in that** the dyestuff is present in a content ranging from 0.001% to 10% by weight relative to the total weight of the composition.

18. Use according to any one of the preceding claims, **characterized in that** the solvent medium comprises at least one organic solvent.

19. Use according to any one of the preceding claims, **characterized in that** the composition also comprises at least one plasticizer.

20. Use according to any one of the preceding claims, **characterized in that** the composition is free of silicone drying agent.
